Europäisches Patentamt

European Patent Office

Office européen des brevets

(11) Veröffentlichungsnummer: **0 489 414 A1**

(12) **EUROPÄISCHE PATENTANMELDUNG**

(21) Anmeldenummer: **91120814.8**

(22) Anmeldetag: **03.12.91**

(51) Int. Cl.⁵: **C02F 3/12, B01J 8/22**

(30) Priorität: **03.12.90 DE 4038514**

(43) Veröffentlichungstag der Anmeldung:
**10.06.92 Patentblatt 92/24**

(84) Benannte Vertragsstaaten:
**AT BE CH DE DK ES FR GB GR IT LI LU NL SE**

(71) Anmelder: **Bräutigam, Hans-Jürgen, Dr.**
**Marmstorfer Weg 74**
**W-2100 Hamburg 90(DE)**

(72) Erfinder: **Bräutigam, Hans-Jürgen, Dr.**
**Marmstorfer Weg 74**
**W-2100 Hamburg 90(DE)**

(74) Vertreter: **Schmidt-Bogatzky, Jürgen, Dr. Ing.**
**Warburgstrasse 50**
**W-2000 Hamburg 36(DE)**

(54) Verfahren zur Anreicherung von Wasser mit Gas und Reaktor zur Durchführung des Verfahrens.

(57) Die Erfindung betrifft ein Verfahren zur Anreicherung von Wasser mit Gas, bei dem das Wasser allgemein senkrecht nach unten strömt und dem Wasser hierbei Gas zugeführt wird und einen Reaktor zur Durchführung des Verfahrens. Das Wasser wird hierbei durch ein Schwebebett (18) aus sich frei bewegenden Partikeln (7) geleitet, die von den eingetragenen Gasblasen durchsetzt sind. Der Gaseintrag kann über eine Gaszuführleitung (11) oder aber eine Membran (12) erfolgen.

Fig.1

Die Erfindung betrifft ein Verfahren zur Anreicherung von Wasser mit Gas, bei dem das Wasser allgemein senkrecht nach unten strömt und dem Wasser hierbei Gas zugeführt wird, wobei in dem Wasser Füllkörper von Wasser und Gas umströmt werden, und einen Reaktor zur Durchführung des Verfahrens.

Es ist bekannt, zur Erhöhung der Gaskonzentration von Wasser dieses in einem geschlossenen U-förmigen Rohr erst nach unten und dann wieder nach oben zu führen, und Gas wie z. B. Sauerstoff, Luft od. dgl. einzuspeisen. Dieses Gas soll in dem Wasser in Lösung gehen. Das gasangereicherte Wasser wird anschließend nachgeschalteten Reaktoren zugeführt, in denen biologische oder physikalisch-chemische Reaktionen durchgeführt werden.

Für eine wirtschaftliche Gasanreicherung müssen Wasserstrom und eingeblasene Gasmenge so aufeinander abgestimmt werden, daß die erzeugten Gasblasen möglichst im Wasserstrom schweben und durch aufsteigende Blasen keine hydraulischen Störungen auftreten bzw. durch absinkende Blasen keine Gasverluste entstehen. Die Aufrechterhaltung dieses empfindlichen Gleichgewichts führte zu technisch aufwendigen Reaktorkonstruktionen und Gasrezirkulation mit Hilfe von Pumpen.

Die Aufgabe der Erfindung besteht darin, das eingangs genannte Verfahren so zu verbessern, daß die Betriebssicherheit erhöht wird und zusätzlich noch biologische und physikalisch-chemische Umsetzungen mit dem gelösten Gas im Wasser durchgeführt werden können. Ferner soll ein Reaktor zur Durchführung des Verfahrens geschaffen werden, der bei größerer Leistungsfähigkeit zur Durchführung des Verfahrens einen kompakten Aufbau hat.

Erfindungsgemäß erfolgt die Lösung der Aufgabe bezüglich des Verfahrens durch die kennzeichnenden Merkmale des Anspruchs 1 und bezüglich des Reaktors durch die kennzeichnenden Merkmale des Anspruchs 8. Vorteilhafte Ausgestaltungen der Erfindung werden in den abhängigen Ansprüchen beschrieben.

Nach der Erfindung ist ein senkrecht nach unten durchströmter, mit Wasser gefüllter Reaktor vorgesehen, in dem sich feste Partikel befinden, die sich aufgrund ihrer im Vergleich zu Wasser geringeren Dichte und der Wasserströmung frei schwebend bewegen. Das zu lösende Gas kann an beliebiger Stelle des Reaktors und des aus den Partikeln gebildeten Schwebebettes eingeblasen werden. Durch den intensiven Kontakt mit den Partikeln wird die Aufenthaltszeit der Blasen im Reaktor stark erhöht und die Stoffaustauschgeschwindigkeit entsprechend gesteigert. Vorteilhaft wirken sich auch die intensiven Partikelbewegungen im Einlaufbereich aus, die Gasblasen zerteilen und deren Wiedervereinigung verhindern.

Der Gaseintrag muß nicht ausschließlich durch Eindüsung erfolgen, sondern kann auch zusätzlich oder allein über gasdurchlässige Schlauch- oder Rohrmembranen aus Siliconkautschuk erfolgen, was an sich bekannt ist. Von besonderem Vorteil ist es jedoch, daß die Kombination der Membranen mit dem erfindungsgemäßen Verfahren erstmals die Möglichkeit bietet, das Leistungspotential der Membranbegasung voll auszuschöpfen, da die an sich unerwünschten Ausgangseffekte (Dissertation H.-J. Bräutigam, 1985, TUHH) bei hohen Drücken nicht stören, sondern hier sehr vorteilhaft zur Erhöhung der Ablaufkonzentration genutzt werden können.

Das erfindungsgemäße Verfahren eignet sich auch für einen effizienten Austrag unerwünschter, gelöster Gase wie z. B. Kohlendioxid, Stickstoff u. dgl. Zu diesem Zweck muß die Gaszufuhr lediglich soweit erhöht werden, daß die Aufnahmekapazität des Wassers überschritten wird. Das überschüssige Gas, in dem die erwünschte Komponente angereichert ist, wird durch den hydrostatischen Druck aus dem Reaktoroberteil, in dem eine Auslaßöffnung wie ein Hahn, Ventil angebracht ist, aus dem Reaktor entfernt. Vorteilhaft bei der Anwendung gaspermeabler Membranen für den kontrollierten Gasaustrag ist, daß die intensive Partikelbewegung leistungsmindernde Ablagerungen auf der Membranoberfläche verhindert.

Erfindungswesentlich ist weiterhin, daß sich in Fortbildung des erfindungsgemäßen Verfahrens auch zusätzlich biologische oder physikalisch-chemisch Umsetzungen durchführen lassen. In diesem Fall ist die Oberfläche der Partikel mit den entsprechenden Mikroorganismen (Nitrifikanten, Denitrifikanten, Essigsäurebakterien etc....) oder Katalysatoren bedeckt, die sich entweder von selbst angesiedelt haben oder künstlich aufgebracht wurden.

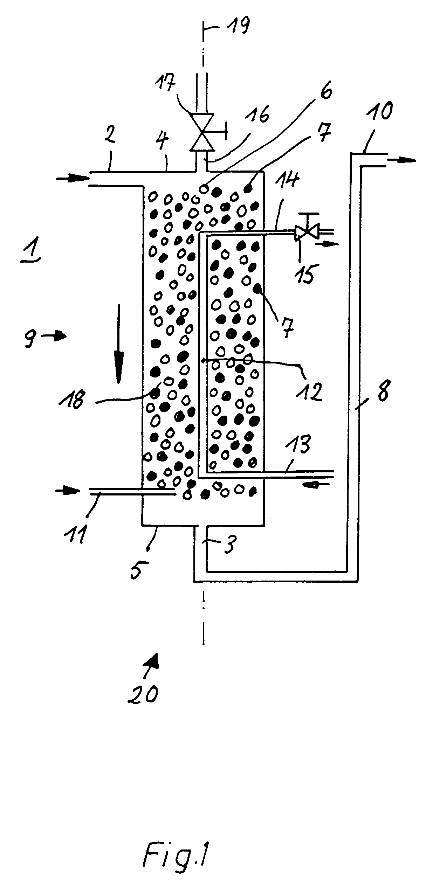

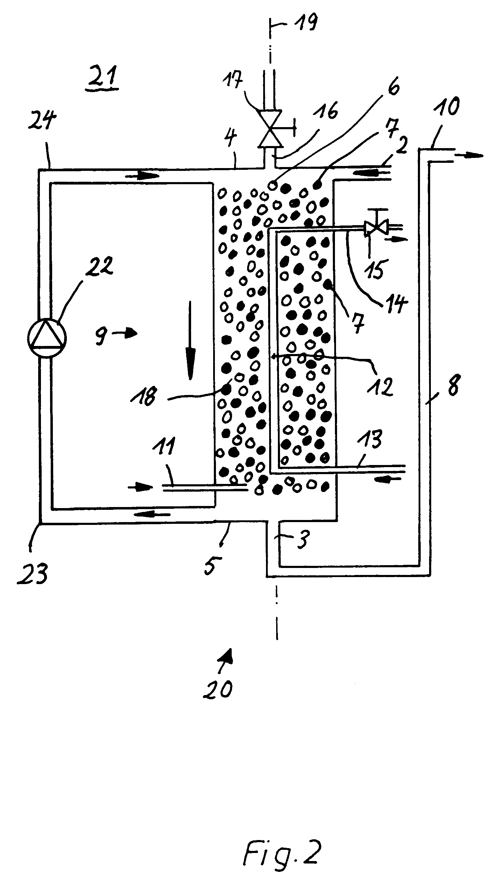

Die Erfindung wird nachstehend am Beispiel der in den Fig. 1 und 2 dargestellten Reaktoren näher erläutert.

Das Fallrohr 9 der U-förmigen Rohranordnung 20 ist als Reaktor 1 ausgebildet. Der Reaktor 1 weist am Reaktorkopf 4 einen Zulauf 2 für Wasser und einen Gasablaßstutzen 16 mit einem Ventil 17 auf. Am Reaktorboden 5 ist der Ablauf 3 für gasangereichertes Wasser vorgesehen, an den das Steigrohr 8 mit dem wasserablauf 10 anschließt. Oberhalb des Reaktorbodens 5 ist eine Gaszuführleitung 11 in den Reaktor 1 eingeführt. In dem Reaktor 1 befindet sich eine Membran 12 aus vorzugsweise Siliconkautschuk, die parallel zur Reaktorlängsachse 19 ausgerichtet ist. Es können auch mehrere Membranen 12 in dem Reaktor angeordnet werden. Die Membran 12 ist mit einer Gaszuführleitung 13 und einer Gasabfuhrleitung 14 mit Ventil 15 verbunden. In dem Reaktor 1 ist ferner ein Schwebebett 18 aus kleinen festen Partikeln 7 ausgebildet.

Der Reaktor 1 wird von oben nach unten von Wasser durchströmt, das über den kopfseitigen Zulauf 2 eintritt und durch einen bodenseitigen Ablauf 3 austritt. Das mit diesem verbundene Steigrohr 8 endet auf dem oder geringfügig über dem Einlaufniveau. In dem Reaktor 1 befinden sich die Partikel 7 frei schwebend und beweglich im Wasserstrom. Zwischen den Partikeln 7 befinden sich Gasblasen 6, die beim Einblasen über die Gaszuführleitung 11 im unteren Teil des Reaktors 1 gebildet werden.

Auf der Oberfläche der Partikel 7 können sich Mikroorganismen oder Katalysatoren befinden, die unter Verbrauch und Nutzung des zugeführten Gases mit Wasserinhaltsstoffen biologische und/oder physikalisch-chemische Umsetzungen durchführen.

Werden nach dem erfindungsgemäßen Verfahren biologische Umsetzungen durchgeführt, muß verhindert werden, daß der biologische Bewuchs auf dem Trägermaterial, der mit der Zeit immer dicker wird, über ein bestimmtes Maß hinaus anwächst. Die Zunahme des Durchmessers führt dazu, daß sich das Schwebebett 18 allmählich nach unten ausdehnt. Des weiteren halten sich die Partikel 7 mit dem größten Durchmesser, d. h. dem dicksten Biofilm in den untersten Bereichen des Schwebebettes 18 auf. Als außerordentlich wirkungsvoll und einfach hat es sich erwiesen, diesen Biofilm mit Hilfe einer Pumpe 22 abzulösen, die saugseitig unterhalb des Schwebebettes 18 mit dem Reaktorunterteil verbunden ist und druckseitig mit dem Reaktorkopf 4.

Ein solcher Reaktor 21 ist in Fig. 2 dargestellt. Die Druckleitung 24 der Pumpe 22 ist im Bereich des Reaktorkopfes 4 und die Saugleitung 23 im Bereich des Reaktorbodens 5 mit dem Fallrohr 9 verbunden.

Die Scherkräfte, die bei Betrieb in der Pumpe 22 auftreten, bewirken, daß überschüssiger Biofilm von den Partikeln 7 abgetrennt wird und als Schwebstoff im Wasser erscheint. Das schwebstoffhaltige Wasser-Partikelgemisch wird in das Reaktoroberteil gefördert.

Die Schwebstoffe, d. h. überschüssige Biomasse, gelangen mit dem Wasserstrom in den Ablauf 3 des Reaktors 21 und können, falls erforderlich, mit bekannten Verfahrenstechniken wie z. B. Flotation, Sedimentation usw. vom Wasser abgetrennt werden.

Die Leistungsfähigkeit des Reaktors 1 zur Durchführung des Verfahrens zur Anreicherung von Wasser mit Gas verdeutlichen folgende Beispiele.

Beispiel 1

In einem Reaktor 1 mit folgenden Abmessungen

- Innendurchmesser 0,12 m
- Höhe 3 m
- Partikeldurchmesser ca. 6 mm
- Partikelmenge ca. 12 l
- Partikelmaterial
  Kunststoffgranulat aus Styrolpolymerisat bzw. Styrolcopolymerisat Styropor

konnte bei einem Wasserdurchsatz zwischen 4 und 8 $m^3/h$ durch Einblasen von Sauerstoff die Sauerstoffkonzentration von 4 g $O_2/m^3$ auf 30 g $O_2/m^3$ erhöht werden.

Beispiel 2

Beim Betrieb einer Versuchsanlage bildete sich bei der Beschickung mit ammoniumhaltigem Wasser auf der Oberfläche der eingesetzten Partikel 7 mit einem mittleren Durchmesser von 1,8 mm ein nitrifizierender Biofilm mit einer Umsatzleistung von 30 $mg^N/m^2$ h. Entsprechend konnte eine auf das Reaktorvolumen bezogene Leistung von 1,5 kg N/$m^3$ x Tag erzielt werden.

**Patentansprüche**

1. Verfahren zur Anreicherung von Wasser mit Gas, bei dem das Wasser allgemein senkrecht nach unten strömt und dem Wasser hierbei Gas zugeführt wird, wobei in dem Wasser Füllkörper von Wasser und Gas umströmt werden, dadurch gekennzeichnet, daß das Wasser durch ein Schwebebett sich frei bewegender Partikel geleitet wird, die von den eingetragenen Gasblasen durchsetzt sind.

2. Verfahren nach Anspruch 1 zur Entfernung unerwünschter Gase oder gasförmiger flüchtiger Komponenten, dadurch gekennzeichnet, daß dem Wasser Gas im Überschuß zugeführt und im Wasser aufkonzentrierte aufsteigende Gasblasen abgeführt werden.

3. Verfahren nach Anspruch 1, dadurch gekennzeichnet, daß dem Schwebebett das einzutragende Gas als Gasstrom zugeführt wird, der sich in und/oder vor Eintritt in das Schwebebett in Gasblasen aufteilt.

4. Verfahren nach Anspruch 1, dadurch gekennzeichnet, daß dem Schwebebett in das Wasser einzutragendes Gas über mindestens eine gasdurchlässige Membran zugeführt wird.

5. Verfahren nach Anspruch 1, 3 und 4, dadurch gekennzeichnet, daß dem Schwebebett das in das Wasser einzutragende Gas durch einen Gasstrom und durch mindestens eine Membran zugeführt wird.

6. Verfahren nach Anspruch 4 und 5, dadurch gekennzeichnet, daß das in das Wasser einzubringende Gas durch eine vom Schwebebett umgebene Membran eingeführt wird.

7. Verfahren nach einem der Ansprüche 1 bis 6, dadurch gekennzeichnet, daß das in das Wasser eingeführte Gas Mikroorganismen oder Katalysatoren zur Durchführung biologischer oder physikalisch-chemischer Umsetzungen mit Wasserinhaltsstoffen zugeführt wird, die im Bereich des Schwebebettes auf den Oberflächen der Partikel des Schwebebettes angelagertsind

8. Reaktor zur Durchführung des Verfahrens nach Anspruch 1 bis 7, gekennzeichnet durch eine von Wasser durchströmte U-förmige Rohranordnung (20), bei der das Fallrohr (9) als Reaktor (1) mit gegenüber dem Zulauf (2), Ablauf (3) und Steigrohr (8) vergrößertem Durchmesser ausgebildet ist, in dem ein von Wasser und Gas durchströmbares Schwebebett (18) aus kleinen festen Partikeln (7) angeordnet ist, und der eine Gaszuführeinrichtung aufweist.

9. Reaktor nach Anspruch 8, dadurch gekennzeichnet, daß die Gaszuführeinrichtung als Gaszuführleitung (11) ausgebildet ist, deren Gasausgang bis unter das Schwebebett (18) geführt ist.

10. Reaktor nach Anspruch 8, dadurch gekennzeichnet, daß die Gaszuführeinrichtung als gasdurchlässige Membran (12) ausgebildet ist, die in dem Schwebebett (18) angeordnet und mit einer Gaszuführleitung (13) und einer Gasabfuhrleitung (14) verbunden ist.

11. Reaktor nach einem der Ansprüche 8 bis 10, dadurch gekennzeichnet, daß auf der Oberfläche der Partikel (7) Mikroorganismen oder Katalysatoren angeordnet sind.

12. Reaktor nach Anspruch 11, mit einer Einrichtung zur Abtrennung überschüssigen biologischen Bewuchses, dadurch gekennzeichnet, daß eine Pumpe (22) saugseitig mittels einer Saugleitung (23) mit dem Reaktorunterteil unterhalb des Schwebebettes (18) und druckseitig mittels einer Druckleitung (24) mit dem Reaktorkopf (4) verbunden ist.

Fig.1

Fig. 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | FR-A-2 371 948 (LINKROSE LIMITED)<br>* Seite 16; Ansprüche 1,3-7 *<br>* Seite 5, Zeile 7 - Seite 7, Zeile 8 *<br>--- | 1,2,7 | C02F3/12<br>B01J8/22 |
| X | DE-A-2 841 011 (METALLGESELLSCHAFT)<br><br>* Seite 1; Ansprüche 1,2,6,7 *<br>* Seite 6, Zeile 17 - Seite 8, Zeile 3 *<br>--- | 1,3,7-9,<br>11,12 | |
| X | DE-A-3 742 642 (BRÄUTIGAM, HANS-JÜRGEN)<br>* Spalte 4; Ansprüche 1,5,6,10,11 *<br>* Spalte 3, Zeile 45 - Spalte 4, Zeile 3 *<br>--- | 1,4-6,10 | |
| X | EP-A-0 025 309 (ECOLOTROL)<br><br>* Seite 19; Ansprüche 1,8,9; Abbildung 4 *<br>--- | 1,7,9,<br>11,12 | |
| A | WO-A-8 700 517 (BRÄUTIGAM, HANS-JÜRGEN ET AL.)<br><br>* Titelseite, Zusammenfassung *<br>* Abbildung 1 *<br><br>----- | 1,3-8,<br>10-12 | |

| RECHERCHIERTE SACHGEBIETE (Int. Cl.5) |
|---|
| C02F<br>B01J |

Der vorliegende Recherchenbericht wurde für alle Patentansprüche erstellt

| Recherchenort | Abschlußdatum der Recherche | Prüfer |
|---|---|---|
| DEN HAAG | 13 MAERZ 1992 | TEPLY J. |

KATEGORIE DER GENANNTEN DOKUMENTE

X : von besonderer Bedeutung allein betrachtet
Y : von besonderer Bedeutung in Verbindung mit einer
   anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
   nach dem Anmeldedatum veröffentlicht worden ist
D : in der Anmeldung angeführtes Dokument
L : aus andern Gründen angeführtes Dokument
........................................................................................
& : Mitglied der gleichen Patentfamilie, übereinstimmendes
   Dokument

EPO FORM 1503 03.82 (P0403)